# EUROPEAN PATENT SPECIFICATION

(11) **EP 2 857 061 B1**
(45) Date of publication and mention of the grant of the patent: **01.02.2017**
(21) Application number: 14173746.0
(22) Date of filing: 24.06.2014
(51) Int. Cl.: A61M 25/09, B21F 7/00

(54) **Shaft and guidewire employing the same**
Welle und Führungsdraht damit
Arbre et fil-guide utilisant celui-ci

(30) Priority: 02.10.2013 JP 2013207156
(43) Date of publication of application: 08.04.2015
(73) Proprietor: ASAHI INTECC CO., LTD., Nagoya-shi, Aichi 463-0024 (JP)
(72) Inventor: Miyata, Naohiko, Nagoya-shi, Aichi 463-0024 (JP); Furukawa, Muneya, Nagoya-shi, Aichi 463-0024 (JP); Matsuo, Kenichi, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 163 276
- US-A- 513 967
- US-A- 5 299 580
- US-A1- 2004 142 643
- US-A1- 2004 215 109

## Description

### Technical Field

The present invention relates to a shaft employed for a medical apparatus inserted into a body cavity for the purpose of treatment or an exam, and a guidewire employing the shaft.

### Background Art

Conventionally, various medical apparatuses inserted into a tubular organ and body tissue such as a blood vessel, a digestive tract and ureter have been proposed for the purpose of using for treatment or an exam.

For example, Patent Literature 1 discloses a guidewire including a shaft twisted around a long axis with the features of the preamble of claim 1.

### Citation List

### Patent Literature

Patent Document 1: U.S. Patent Application No. 2004/0215109

### Summary of Invention

### Technical Problem

In the case of inserting a conventionally known guidewire along an inverted U-shaped path from the lower extremity vasculature of the right leg into the lower extremity vasculature of the left leg by, for example, the Cross-Over method, in such the extremely winding lower extremity vasculature, a shaft configuring the guidewire possibly bends excessively due to a load applied thereto when coming into contact with a blood vessel wall or the like.

Accordingly, a permanent set (plastic deformation) occurs in the shaft, thereby possibly interfering with subsequent operation.

The present invention has been devised in view of such circumstances, and it is an object of the present invention to provide a shaft capable of suppressing a permanent set even when inserted into an extremely winding blood vessel, and a guidewire employing the shaft.

### Solution to Problem

In order to solve the above-described problem, a shaft according to one aspect of the present invention and a guidewire employing the shaft have features below.

A shaft according to a first aspect of the present invention is a shaft twisted along a longitudinal direction, having a cross section taking the form of an approximately rectangular shape in a direction vertical to the longitudinal direction, in which the cross section has a pair of recesses recessed arcuately in a direction vertical to the longitudinal direction and having the recesses formed on a pair of sides facing each other among sides forming the cross section.

A second aspect of the present invention is the shaft according to the first aspect having projections projected arcuately on a pair of sides facing each other except those having the recesses in a direction vertical to the longitudinal direction.

A third aspect of the present invention is the shaft according to the second aspect having the radius of curvature of the projection smaller than the radius of curvature of a virtual circle having a diameter equivalent to a long axis of the cross section.

A fourth aspect of the present invention is a guidewire including a core shaft and a coiled body covering a distal portion of the core shaft, in which a proximal portion of the core shaft located on a proximal side of the coiled body is the shaft according to any one of the first to third aspects.

A fifth aspect of the present invention is the guidewire according to the fourth aspect having the coiled body composed of a plurality of strands wound helically, each of the strands being made of a core wire and side wires wound so as to cover a circumference of the core wire.

### Advantageous Effects of Invention

The shaft of a guidewire not covered by the claims has a cross section with a recess recessed arcuately. In this manner, in the shaft having a cross section with a recess recessed arcuately, comparing to a configuration without such the recess (for example, a shaft having a cross-sectional rectangular shape), moment of inertia of area is lowered.

Therefore, when allowing the shaft to enter along a path winding in an inverted U-shape from the lower extremity vasculature of the right leg into the lower extremity vasculature of the left leg by, for example, the Cross-Over method, a permanent set tends not to occur in the shaft even when subjected to a load caused by contact with a blood vessel wall or the like to be bent excessively. Thus, there is no possible trouble in subsequent operation, thereby making it possible to use the shaft continuously.

In the shaft of the first aspect, recesses are formed on a pair of sides facing each other among sides forming a cross section. Thereby, comparing to a configuration without such recesses (a shaft having a cross-sectional rectangular shape), moment of inertia of area is still further lowered. Therefore, for example, within a blood vessel of a lower extremity region extremely winding in an inverted U-shape, a permanent set still further tends not to occur in the shaft even when subjected to a load caused by contact with a blood vessel wall or the like to be bent excessively. Thus, there is no possible trouble in subsequent operation, thereby making it possible to use the shaft continuously.

The shaft of the second aspect has projections projected arcuately on a pair of sides facing each other except those having recesses, and top parts of the projected projections come into contact with a blood vessel wall. That is, comparing to a configuration without such the projections (a configuration having a cross-sectional rectangular shape whose four corners are in contact with a blood vessel wall), in the shaft of the present invention, contact parts with a blood vessel wall are reduced as much as possible, while a load applied to the blood vessel wall in contact also gets smaller.

Accordingly, operation resistance of the shaft pushed and pulled is reduced so that torque transmission characteristics are enhanced, resulting in improved operability. That is, even in a case where the above shaft is inserted along an inverted U-shaped path from the lower extremity vasculature of the right leg into the lower extremity vasculature of the left leg by, for example, the Cross-Over method, when passing through the top of the path, sliding against the blood vessel wall and the like do not cause movement of the shaft to be suppressed, so that a distal portion of the shaft is allowed to be inserted deeply and smoothly into the path. In addition, it is possible to reduce damage of a blood vessel.

In the shaft of the third aspect, the radius of curvature of a projection is smaller than the radius of curvature of a virtual circle whose diameter is equivalent to a long axis of a cross section of the shaft. Such the shaft has a cross section in a tapered shape, and has two tops of respective projections in contact with a blood vessel wall. Therefore, comparing to a configuration without such the projection (a configuration having a cross-sectional rectangular shape whose four corners are in contact with a blood vessel wall), in the shaft of the fourth aspect, contact areas (contact parts) with a blood vessel wall are surely reduced.

Accordingly, when the above shaft rotates and enters the inside of a blood vessel, operation resistance of the shaft pushed and pulled is surely reduced so that sufficient torque transmission characteristics are obtained, resulting in further improved operability. Furthermore, it is possible to reduce damage of a blood vessel as much as possible.

The guidewire of the fourth aspect includes a core shaft and a coiled body covering a distal portion of the core shaft, in which a proximal portion of the core shaft located on a proximal side of the coiled body is the shaft according to any one of the first to fourth aspects. Therefore, it is possible to obtain the above-described effect according to the first to third aspects. That is, in the case of allowing the guidewire to enter along a path extremely winding in an inverted U-shape, a permanent set tends not to occur in the shaft even when subjected to a load caused by contact with a blood vessel wall or the like to be bent excessively.

Further, it is possible to insert a distal portion of the guidewire even into an extremely winding blood vessel in an inverted U-shape deeply and smoothly. Moreover, operation resistance of the guidewire pushed and pulled is further reduced, while making it possible to reduce damage of a blood vessel effectively.

The guidewire of the fifth aspect includes a coiled body composed of a plurality of strands wound helically, each of the strands being made of a core wire and side wires wound so as to cover a circumference of the core wire. Thereby, comparing to a guidewire including a coiled body composed of, for example, a single wire having an external diameter nearly equal to that of the guidewire of the sixth aspect, flexibility of the coiled body is improved, and it is possible to secure sufficient torque transmission characteristics. Further, breaking strength against twisting is improved, so that safety of the guidewire is also enhanced.

### Brief Description of Drawings

Fig. 1 is a general view showing a shaft of the guidewire.
Fig. 2 is a cross-sectional view showing a shaft.
Fig. 3 is a cross-sectional view showing a shaft of a first embodiment of the present invention.
Fig. 4 is a cross-sectional view showing a shaft of a second embodiment of the present invention.
Fig. 5 is a cross-sectional view showing a shaft of a third embodiment of the present invention.
Fig. 6 is a general view showing a first embodiment of a guidewire of the present invention.
Fig. 7 is a general view showing a second embodiment of a guidewire of the present invention.

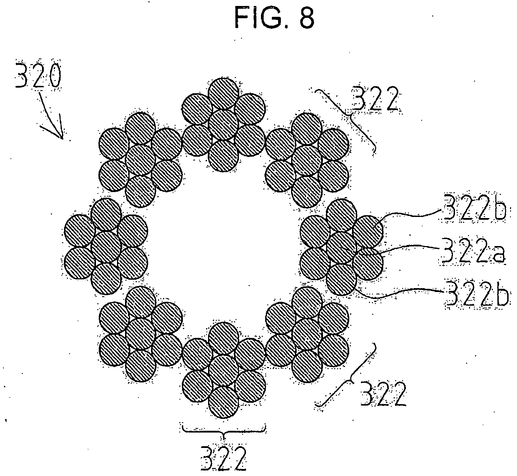

Fig. 8 is a cross-sectional view along A-A of a first coiled body in Fig. 7.

### Description of Embodiments

First, description will be given for a shaft of the present invention based on preferred embodiments shown in the drawings.

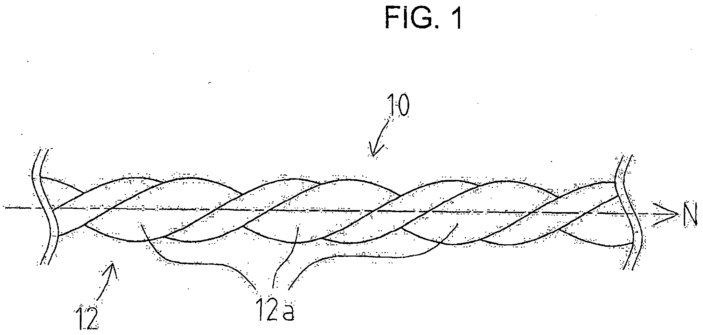

Fig. 1 is a general view showing a shaft. Note that, in this figure, a length direction of the shaft is shortened to be schematically illustrated in whole in order to facilitate the understanding, thus providing an overall dimension different from the actual one.

As shown in Fig. 1, a shaft 10 takes on a rod-like body in an elongated shape. The shaft 10 can be formed using materials such as, for example, stainless steel (SUS304), a super elastic alloy such as an Ni-Ti alloy, and a piano wire, but not especially limited thereto.

The shaft 10 is twisted in a predetermined direction along a longitudinal direction N, and has a helically shaped portion 12. Additionally, the shaft 10 is provided with a plurality of grooves 12a at regular intervals along the longitudinal direction N. Thereby, for example, when inserting the shaft 10 into a blood vessel, the plurality of grooves 12a leads to reduction of contact areas with a blood vessel wall.

Further, the shaft 10 has the helically shaped portion 12, and when a proximal side of the shaft 10 is rotated, such rotation is thereby easily transmitted to a distal side of the shaft 10. That is, torque transmission characteristics are enhanced, resulting in improved operability.

Moreover, in Fig. 1, a direction of helix of the helically shaped portion 12 is counterclockwise along the longitudinal direction N of the shaft 10. However, the direction of helix of the helically shaped portion 12 is not limited thereto, and may be clockwise along the longitudinal direction N of the shaft 10.

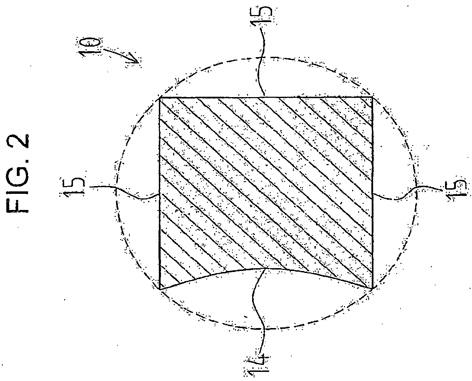

As shown in Fig. 2, the shaft 10 has a cross section in an approximately rectangular shape in a direction vertical to the longitudinal direction N (hereinafter, simply referred to as a cross section). Further, the cross section has a recess 14 recessed arcuately. In the present example, the recess 14 is provided only on one side among four sides forming the cross section. Moreover, the other three sides are linear portions 15 formed linearly.

In the exemple in which the recess 14 is provided on one side among sides forming a cross section, comparing to a configuration without any such the recess 14 (a shaft having a cross-sectional rectangular shape), moment of inertia of area of the shaft 10 is lowered. Thereby, when allowing the shaft to enter along a path winding in an inverted U-shape from the lower extremity vasculature of the right leg into the lower extremity vasculature of the left leg by, for example, the Cross-Over method, a permanent set tends not to occur in the shaft even when subjected to a load caused by contact with a blood vessel wall or the like to be bent excessively. Therefore, there is no possible trouble in subsequent operation, thereby making it possible to use the shaft 10 continuously.

Here, it is possible to fabricate the shaft 10 according to a method described below. First, a columnar metallic body is prepared to be rolled out from a predetermined direction. Thereafter, a rotary device is prepared, capable of applying rotary motion to a circumference of the metallic body having one end fixed, followed by rotation around a long axis of the metallic body from the other end.

Further, a distal end of the metallic body is fixed to one end of the rotary device while a proximal end of the metallic body is fixed to the other end of the rotary device, thereafter applying rotary motion from the other end of the rotary device, so that the metallic body is twisted from the proximal side. Thereby, the shaft 10 is formed, including the helically shaped portion 12 twisted evenly along the longitudinal direction N.

The helically shaped portion 12 is twisted by the rotary device, and thereafter has stress applied by the twisting mitigated by means of heat treatment, thereby taking the form of a stable shape. A method of forming the helically shaped portion 12 is not limited to the above-described method, and may be fabricated by the other known method adopted appropriately.

### First Embodiment:

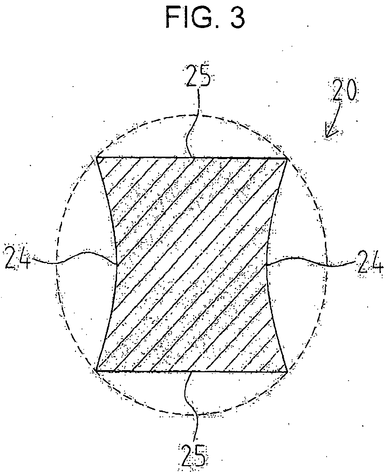

Fig. 3 is a cross-sectional view showing a first embodiment of the shaft of the present invention. Note that, in this figure, a cross section of the shaft is schematically illustrated, thus having a dimensional ratio different from the actual one.

In the shaft 10 of the above-described example, the recess 14 is provided only on one side among sides forming a cross section, and linear portions 15 are provided on the other sides. Whereas, in a shaft 20 of the first embodiment, recesses 24 are formed on a pair of sides facing each other. On sides except those having the pair of the recesses 24, linear portions 25 are provided, respectively.

Thereby, comparing to not only a configuration without a recess (a shaft having a cross-sectional rectangular shape) but also the example in which the recess 14 is provided only on one side among sides forming a cross section, moment of inertia of area is still further lowered.

Therefore, for example, within a blood vessel of a lower extremity region extremely winding in an inverted U-shape, a permanent set still further tends not to occur in the shaft 20 even when subjected to a load caused by contact with a blood vessel wall or the like to be bent excessively. Thus, there is no possible trouble in subsequent operation, thereby making it easy to use the shaft 20 continuously.

### Second Embodiment

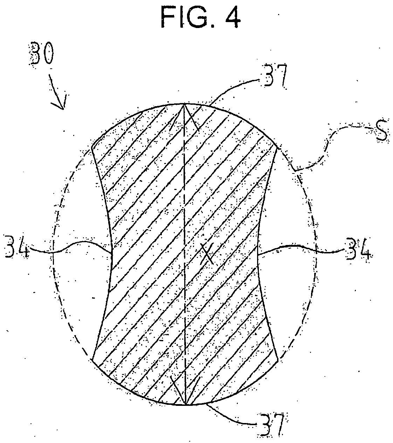

Fig. 4 is a cross-sectional view showing a second embodiment of the shaft of the present invention. Note that, in this figure, a cross section of the shaft is schematically illustrated, thus having a dimensional ratio different from the actual one.

In the shaft 20 of the above-described embodiment, the recesses 24 are formed on a pair of sides facing each other, and the linear portions 25 are provided respectively on sides except those having the pair of the recesses 24. Whereas, in a shaft 30 of the second embodiment, on sides except those having a pair of recesses 34 among sides forming a cross section, projections 37 projected arcuately are provided, respectively. Note that, in the present embodiment, the radius of curvature of the projection 37 is set so as to be approximately the same as the radius of curvature of a virtual circle S having a diameter equivalent to a long axis X of the cross section.

Thereby, when inserting the shaft 30 into a blood vessel, top parts of the projections 37 provided on a pair of sides only come into contact with a blood vessel wall. That is, comparing to a configuration without such the projections 37 (for example, a configuration having a cross-sectional rectangular shape whose four corners are in contact with a blood vessel wall), in the shaft 30 of the present embodiment, contact parts with a blood vessel wall are surely reduced, while a load applied to the blood vessel wall in contact is also still further smaller.

Therefore, when allowing the shaft 30 to rotate and enter the inside of a blood vessel, operation resistance against a blood vessel wall is surely reduced. Accordingly, operation resistance of the shaft 30 pushed and pulled is surely reduced so that sufficient torque transmission characteristics are obtained, resulting in enhanced operability.

Moreover, even in a case where the above shaft 30 is inserted along an inverted U-shaped path from the lower extremity vasculature of the right leg into the lower extremity vasculature of the left leg by, for example, the Cross-Over method, when passing through the top of the path, sliding against the blood vessel wall and the like do not cause movement of the shaft 30 to be suppressed, so that a distal portion of the shaft is allowed to be inserted deeply and smoothly into the path. In addition, it is possible to reduce damage of a blood vessel.

### Third Embodiment

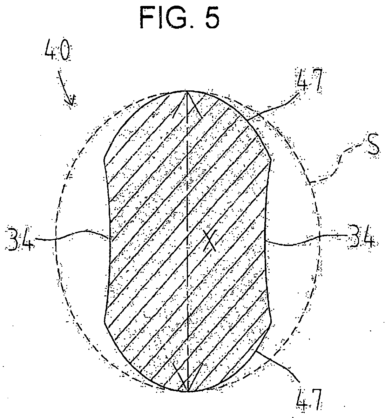

Fig. 5 is a cross-sectional view showing a third embodiment of the shaft of the present invention. Note that, in this figure, a cross section of the shaft is schematically illustrated, thus having a dimensional ratio different from the actual one.

In the above-described second embodiment, the projections 37 projected arcuately are provided respectively on sides except those having a pair of the recesses 34, and the radius of curvature of these projections 37 is set so as to be approximately the same as the radius of curvature of the virtual circle S having a diameter equivalent to the long axis X of the cross section. Whereas, in a cross section forming a shaft 40 of the present embodiment, the radius of curvature of a pair of projections 47 is smaller than the radius of curvature of the virtual circle S having a diameter equivalent to the long axis X of the cross section.

Such the shaft 40 has a cross section in a tapered shape, and has two tops of respective projections 47 in contact with a blood vessel wall. Therefore, comparing to not only a configuration without a projection (a configuration having a cross-sectional rectangular shape whose four corners are in contact with a blood vessel wall) but also the third embodiment in which a projection is provided having the radius of curvature approximately the same as the radius of curvature of the virtual circle S, in the shaft 40 of the present embodiment, contact areas (contact parts) with a blood vessel wall are more surely reduced.

Accordingly, when the shaft 40 rotates and enters the inside of a blood vessel, operation resistance of the shaft 40 pushed and pulled is surely reduced so that sufficient torque transmission characteristics are secured, resulting in still further enhanced operability. Furthermore, it is possible to surely reduce damage of a blood vessel.

### Fourth Embodiment

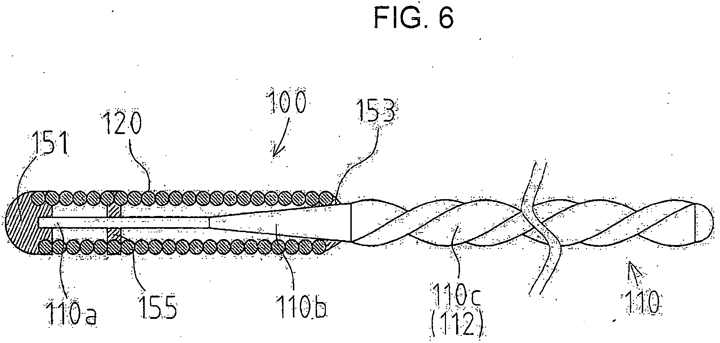

Fig. 6 is a general view showing a first embodiment of a guidewire of the present invention. In Fig. 6, a distal side of the guidewire to be inserted into the body is provided on the left, and a proximal side of the guidewire to be operated by a manipulator such as a doctor is provided on the right. Note that, in this figure, the guidewire is schematically illustrated; thus having a dimensional ratio different from the actual one.

A guidewire 100 shown in Fig. 6 is used for treatment of the lower extremity vasculature by, for example, the Cross-Over method. The guidewire 100 includes a core shaft 110, and a coiled body 120 covering a circumference of a distal portion of the core shaft 110.

First, description will be given for the core shaft 110. The core shaft 110 includes a small diameter portion 110a, a tapered portion 110b and a large diameter portion 110c in order from the distal side to the proximal side. The small diameter portion 110a is a part on the most distal side of the core shaft 110 and the most flexible part of the core shaft 110. The small diameter portion 110a is formed in a tabular shape by press working. The tapered portion 110b has a cross section formed in a tapered round shape whose diameter is gradually reduced toward the distal side. Note that, arrangements and dimensions of the small diameter portion 110a and the tapered portion 110b can be changed appropriately due to reasons to obtain desired rigidity and the like. For example, the small diameter portion 110a may have a columnar shape. Further, the number of the tapered portions 110b and the angle of the tapered portion 110b may also be set appropriately as necessary.

The large diameter portion 110c is located on a proximal side of the coiled body 120, and takes on a shape similar to that of the shaft in the above-described third embodiment. That is, the proximal side exposed from the coiled body 120 in the core shaft 110 is twisted along a longitudinal direction, and has a helically shaped portion 112. Moreover, as described in the third embodiment, a cross section of the helically shaped portion 112 has a recess 34 recessed arcuately.

Thereby, comparing to a configuration without any such the recess 34 (a shaft having a cross-sectional rectangular shape), moment of inertia of area of the shaft 10 is further lowered. Thereby, when allowing the guidewire 100 to enter along a path winding in an inverted U-shape from the lower extremity vasculature of the right leg into the lower extremity vasculature of the left leg by, for example, the Cross-Over method, a permanent set tends not to occur in the guidewire 100 even when subjected to a load caused by contact with a blood vessel wall or the like to be bent excessively. Therefore, there is no possible trouble in subsequent operation, thereby making it possible to use the guidewire 100 continuously.

Note that, in the guidewire 100 of the present embodiment, the large diameter portion 110c having the same shape as that of the shaft of the above-described fourth embodiment is adopted, however, the shape of the large diameter portion 110c is not limited thereto. That is, a large diameter portion may be adopted having the same shape as that of the shaft of any one of the first to second embodiments. Even in this case, the same effect as that of the present embodiment is provided.

The core shaft 110 can be formed using materials such as, for example, stainless steel (SUS304), a super elastic alloy such as an Ni-Ti alloy, and a piano wire, however, not especially limited thereto.

Next, description will be given for the coiled body 120. The coiled body 120 in the present embodiment is a single thread coil composed of wires wound helically.

As shown in Fig. 6, a distal end of the coiled body 120 is fixed a distal end of the core shaft 110 with a distal side joint 151. A proximal end of the coiled body 120 is fixed the core shaft 110 with a proximal side joint 153. Further, an approximately middle part of the coiled body 120 located on a distal side from the proximal side joint 153 and on a proximal side from the distal side joint 151 is fixed the core shaft 110 with a middle joint 155.

Materials forming the distal side joint 151, the proximal side joint 153 and the middle joint 155 are not especially limited, but include, for example, brazing metal such as an Sn-Pb alloy, a Pb-Ag alloy, an Sn-Ag alloy and an Au-Sn alloy.

Materials forming the coiled body 120 are not especially limited, but can employ a radiopaque wire or a radiolucent wire. Materials used for a radiopaque wire are not especially limited, but can include, for example, gold, platinum, tungsten, an alloy containing these elements (for example, a platinum-nickel alloy), or the like. Moreover, materials used for a radiolucent wire are not especially limited, but can include, for example, stainless steel (SUS304, SUS316 and the like), a super elastic alloy such as an Ni-Ti alloy, a piano wire, and the like.

### Fifth Embodiment

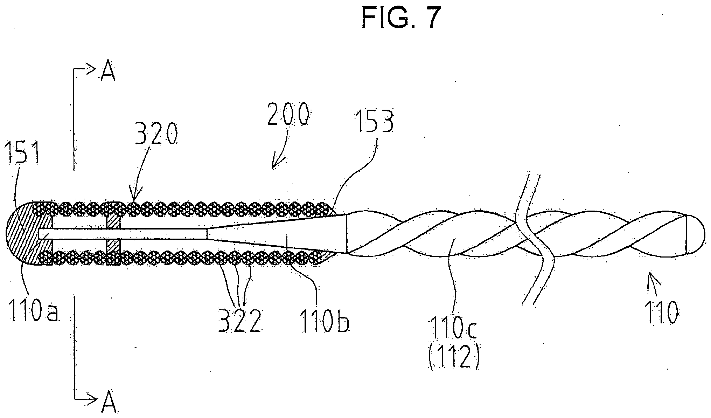

Fig. 7 is a general view showing a second embodiment of a guidewire of the present invention. In Fig. 7, a distal side of the guidewire to be inserted into the body is provided on the left, and a proximal side of the guidewire to be operated by a manipulator such as a doctor is provided on the right. Note that, in this figure, the guidewire is schematically illustrated, thus having a dimensional ratio different from the actual one.

A guidewire 200 of the present embodiment has a configuration of a coiled body different from that of the above-described fourth embodiment. That is, a coiled body 320 employed in the guidewire 200 of the present embodiment is composed of a plurality of strands 322 (eight strands in the present embodiment) wound helically, the strand 322 being made of a core wire (wire) 322a and six side lines (wires) 322b wound so as to cover a circumference of the core wire 322a. Materials forming the core wire 322a and the side line 322b are not especially limited, but include, for example, stainless steel, tungsten, an Ni-Ti alloy and the like.

According to the guidewire 200 of the present embodiment, comparing to a guidewire including a coiled body composed of, for example, a single wire having an external diameter nearly equal to that of the guidewire 200, flexibility of the coiled body is improved, thereby making it possible to secure sufficient torque transmission characteristics. Further, breaking strength against twisting is improved, so that safety of the guidewire 200 is also enhanced.

### References Signs List

10, 20, 30, 40...shaft; 14,24,34...recess; 37, 47...projection; 100,200...guidewire; 110...core shaft; 120, 320...coiled body; 322...strand; N...longitudinal direction; X...long axis of a cross section; and S...virtual circle.

## Claims

1. A shaft (20, 30, 40) twisted along a longitudinal direction, having a cross section taking the form of an approximately rectangular shape in a direction vertical to the longitudinal direction, **characterized in that**
the cross section has a pair of recesses (24, 34) recessed arcuately in a direction vertical to the longitudinal direction, and
the recesses (24, 34) are formed on a pair of sides facing each other among sides forming the cross section.

2. The shaft according to claim 1, comprising
projections (37, 47) projected arcuately on a pair of sides facing each other except those having the recesses in the direction vertical to the longitudinal direction.

3. The shaft according to claim 2, wherein
the radius of curvature of the projections (47) is smaller than the radius of curvature of a virtual circle having a diameter equivalent to a long axis of the cross section.

4. A guidewire comprising:
a core shaft (110); and
a coiled body (120, 320) covering a distal portion of the core shaft, wherein
a proximal portion (110c) of the core shaft located on a proximal side of the coiled body is the shaft according to any one of claims 1 to 3.

5. The guidewire according to claim 4, wherein
the coiled body (320) is composed of a plurality of strands wound helically, each of the strands being made of a core wire and side wires wound so as to cover a circumference of the core wire.

## Patentansprüche

1. Schaft (20, 30, 40), der längs verdrillt ist und in einer Richtung quer zur Längsrichtung einen in etwa rechteckförmigen Querschnitt hat, **dadurch gekennzeichnet, dass**
der Querschnitt zwei Aussparungen (24, 34) aufweist, die in einer Richtung quer zur Längsrichtung bogenförmig ausgespart sind, und
die Aussparungen (24, 34) an zwei einander gegenüberliegenden Seiten der den Querschnitt formenden Seiten gebildet sind.

2. Schaft nach Anspruch 1, mit
Vorsprüngen (37, 47), die an zwei einander gegenüberliegenden anderen Seiten als den Seiten mit den Aussparungen in der Richtung quer zur Längsrichtung bogenförmig vorspringen.

3. Schaft nach Anspruch 2, wobei
der Krümmungsradius der Vorsprünge (47) kleiner ist als der Krümmungsradius eines virtuellen Kreises mit einem Durchmesser gleich einer Längsachse des Querschnitts.

4. Führungsdraht mit:
einem Kernschaft (110) ; und
einem Wicklungskörper (120, 320), der einen distalen Abschnitt des Kernschafts umgibt, wobei
ein auf einer proximalen Seite des Wicklungskörpers liegender proximaler Abschnitt (110c) des Kernschafts ein Schaft nach einem der Ansprüche 1 bis 3 ist.

5. Führungsdraht nach Anspruch 4, wobei
der Wicklungskörper (320) aus einer Vielzahl von schraubenförmig gewickelten Strängen besteht, die jeweils aus einem Kerndraht und um den Umfang des Kerndrahts herum gewickelten Seitendrähten gebildet sind.

## Revendications

1. Arbre (20, 30, 40) torsadé le long d'une direction longitudinale, ayant une section transversale prenant la forme d'une forme approximativement rectangulaire dans une direction verticale par rapport à la direction longitudinale, **caractérisé en ce que**
la section transversale a une paire de renfoncements (24, 34) renfoncés de façon arquée dans une direction verticale par rapport à la direction longitudinale, et
les renfoncements (24, 34) sont formées sur une paire de faces se faisant face l'une à l'autre entre les faces formant la section transversale.

2. Arbre selon la revendication 1, comprenant
des projections (37, 47) projetées de façon arquée sur une paire de faces se faisant face l'une à l'autre sauf celles ayant des renfoncements dans la direction verticale par rapport à la direction longitudinale.

3. Arbre selon la revendication 2, dans lequel
le rayon de courbure des projections (47) est plus petit que le rayon de courbure d'un cercle virtuel ayant un diamètre équivalent à un axe long de la section transversale.

4. Fil de guidage comprenant :
un arbre noyau (110) ; et
un corps embobiné (120, 320) couvrant une portion distale de l'arbre noyau, dans lequel
une portion proximale (110c) de l'arbre noyau située sur un côté proximal du corps embobiné est l'arbre selon l'une quelconque des revendications 1 à 3.

5. Fil de guidage selon la revendication 4, dans lequel
le corps embobiné (320) est composé d'une pluralité de torons enroulés de façon hélicoïdale, chacun des torons étant constitué d'un fil d'âme et de fils latéraux enroulés de façon à couvrir une circonférence du fil d'âme.
